# EUROPEAN PATENT APPLICATION

(11) **EP 4 112 732 A1**
(43) Date of publication of application: **04.01.2023**
(21) Application number: 21761577.2
(22) Date of filing: 26.02.2021
(51) Int. Cl.: C12P 7/26

(54) **NOVEL FLAVONE HYDROXYLASES, MICROORGANISM FOR SYNTHESIZING FLAVONE C-GLYCOSIDE COMPOUNDS, AND USE THEREOF**

(30) Priority: 28.02.2020 CN 202010129425
(71) Applicant: CAS Center for Excellence in Molecular Plant Sciences, Shanghai 200032 (CN)
(72) Inventor: WANG, Yong, Shanghai 200032 (CN); SUN, Yuwei, Shanghai 200032 (CN); CHEN, Zhuo, Shanghai 200032 (CN)
(74) Representative: EP&C
(86) International application number: PCT/CN2021/078193
(87) International publication number: WO 2021/170097

(57) **Abstract**

Provided are novel flavone hydroxylases, a microorganism for synthesizing flavone C-glycoside compounds, and use thereof. The present inventor obtains novel flavones hydroxylates PhF2H and PhF3'H by cloning, which belong to cytochrome P450 hydroxylates and have the function of hydroxylating specific positions of compounds. Further, the present inventor, by modifying the enzymes and combining a C-glycoside glycosyltransferase and assembly of a synthesis pathway of a flavone precursor, efficiently synthesizes flavone C-glycoside compounds such as oriention, isooriention, vitexin and isovitexin, and related intermediates such as eriodictyol and 2-hydroxynaringenin in the synthesis pathway thereof in an artificial recombinant expression system.

## Description

### Technical field

The present disclosure relates to the field of synthetic biology and pharmaceutical technology, in particular, the present disclosure relates to novel flavone hydroxylases, microorganism for synthesizing flavone C-glycoside compounds or intermediates, a preparation method and use thereof.

### Background

Bamboo has been widely used as a food-medicine plant since ancient times. "Bamboo leaves can clear away heat, prevent abortion, produce body fluids and induce diuresis" recorded in *Chinese Dictionary of Herbal Medicine* indicates the edibleness of bamboo leaves; and "bamboo leaves can exert effects in muscles and skin, heal sores and kill insects" recorded in *Wenjing Fengyuan* also indicates the external medicinal properties. Bamboo leaves are widely spread in the south of the Yangtze River as a heat-clearing tea drink. Main flavone extracts in bamboo leaves are four C-glycoside flavone compounds: orientin, isoorientin, vitexin and isovitexin.

C-glycoside flavone compounds are compounds with C6-C3-C6 structure as parent nucleus, which is directly linked by C-C bond to a glycosyl group. C-glycoside flavones are more rare than O-glycoside flavones. So far, there are dozens of C-glycoside flavones isolated from nature. According to the parent structure of flavones, flavones can be divided into C-glycoside flavones, flavonol C-glycoside flavones and di-C-glycoside flavone compounds. Among them, C-glycoside flavone compounds are the most widely distributed. According to the reports, C-glycoside flavone compounds have a diversity of functions including significant antioxidation, significant inhibition of exogenous and endogenous free radicals, inhibition of bacteria and viruses in a certain degree, regulation of blood lipids, significant reduction of the content of total cholesterol in the blood, and pharmaceutical activities of treating cardiovascular and cerebrovascular diseases. C-glycoside flavone compounds also have significant anti-radiation and neuro-protective effects. Surveys have shown that C-glycoside flavones have effects on related antimetabolic diseases, and have certain therapeutic effects on diabetes, obesity and other diseases.

Like most natural products, several C-glycoside flavone compounds are very difficult to be prepared through chemical organic synthetic methods. Presently, C-glycoside flavone compounds are mainly obtained by extracting organic reagents from plants. In this process, a large number of organic solvents are required, with subsequent cumbersome isolation processes and following problems such as high costs of industrialization. Moreover, the slow growth of plants is the main bottleneck of this method.

At present, with the development of synthetic biology, by using microorganisms as host cells and introduce the exogenous genes therein, and using microbial reactor fermentation methods to directional synthesize target products is becoming an increasingly-common technology. Microorganisms are superior in simple genetic manipulation and rapid growth. Compared with traditional plant extraction methods, microbial fermentation have several advantages, such as more fast synthesis, less affection by weather and climate, and easier obtainment of some intermediates. Also, the yield of some compounds synthesized by microorganisms is much higher than that of plant extraction. Microbial fermentation has already become an important method of obtaining natural products.

Currently, there are no reports of efficient synthesis of C-glycoside flavone compounds in *E. coli.* Therefore, to explore effectively and find efficient synthetic pathways for such compounds is urgent in this field.

### Summary of disclosure

The present disclosure aims to provide novel flavone hydroxylases, microorganism for synthesizing flavone C-glycoside compounds or intermediates, a preparation method and use thereof.

First aspect of the present disclosure provides a method of catalyzing C-2 position or C-3' position hydroxylation of flavanone (class) compounds, comprising catalyzing with a novel flavone hydroxylase; wherein the novel flavone hydroxylase is a polypeptide shown in SEQ ID NO: 1 or a conservative variant polypeptide thereof, which catalyzes the hydroxylation of flavanone (class) compounds at the C-2 position; or wherein the novel flavone hydroxylase is a polypeptide shown in SEQ ID NO:2 or a conservative variant polypeptide thereof, which catalyzes the hydroxylation of flavanone (class) compounds at the C-3'position.

In a preferable example, the flavanone (class) compound has a parent nucleus structure of formula (I), forms a parent nucleus structure of formula (II) after hydroxylation at the C-2 position, or a parent nucleus structure of formula (III) after hydroxylation at the C-3'position;

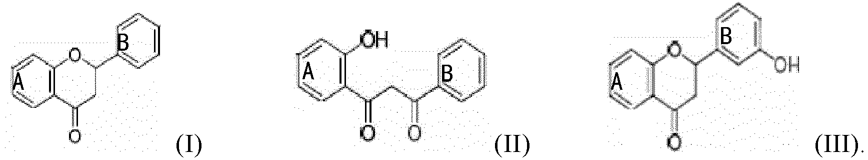

In another preferable example, in A ring or B ring, there are 1, 2 or 3 hydroxyl groups.

In another preferable example, the flavanone (class) compound comprises (but not limited to): naringenin, eriodictyol, pinocembrin, hesperidin, sakuranetin.

In another preferable example, the product of hydroxylation at the C-2 position is a 2-hydroxy flavanone compound, comprising (but not limited to): 2-hydroxynaringenin, 2-hydroxyeriodictyol, 2-hydroxypinocembrin, 2-hydroxyhesperidin, 2-hydroxysakuranetin; after hydroxylation at the C-2 position of the flavanone (class) compound, the 2-hydroxy flavanone compound is formed preferably in an open-loop.

In another preferable example, the product of hydroxylation at the C-3' position is a 3'-hydroxy flavanone compound, comprising (but not limited to): eriodictyol, 3'-hydroxyeriodictyol, 3'-hydroxysakuranetin.

Another aspect of the present disclosure provides a use of a novel flavone hydroxylase for catalyzing C-2 or C-3' position hydroxylation of flavanone (class) compounds. The novel flavone hydroxylase is a polypeptide shown in SEQ ID NO:1 or a conservative variant polypeptide thereof, which catalyzes the hydroxylation of flavanone (class) compounds at the C-2 position. The novel flavone hydroxylase is a polypeptide shown in SEQ ID NO:2 or a conservative variant polypeptide thereof, which catalyzes the hydroxylation of flavanone (class) compounds at the C-3'position.

In a preferable example, the flavanone (class) compound has a parent nucleus structure of formula (I), a parent nucleus structure of formula (II) (in an open-loop) after hydroxylation at the C-2 position, or a parent nucleus structure of formula (III) after hydroxylation at the C-3'position.

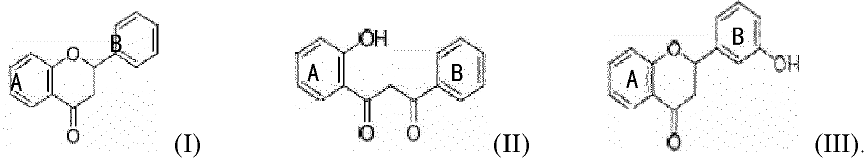

In another preferable example, the flavanone (class) compound comprises (but not limited to): naringenin, eriodictyol, pinocembrin, hesperidin, sakuranetin.

In another preferable example, the product of hydroxylation at the C-2 position comprising (but not limited to): 2-hydroxynaringenin, 2-hydroxyeriodictyol, 2-hydroxypinocembrin, 2-hydroxyhesperidin, 2-hydroxysakuranetin; after hydroxylation at the C-2 position of the flavanone (class) compound, the 2-hydroxy flavanone compound is formed preferably in an open-loop.

In another preferably example, the product of hydroxylation at the C-3' position comprising (but not limited to): eriodictyol, 3'-hydroxyeriodictyol, 3'-hydroxysakuranetin.

In another preferable example, in the polypeptide shown in SEQ ID NO: 1 or a conservative variant polypeptide thereof, N-terminal amino acids of transmembrane region are partially truncated (eg. 5, 10, 15, 18, 20aa or more truncated) or completely truncated; preferably N-terminal amino acids 2nd to 24th are truncated.

In another preferable example, in the polypeptide shown in SEQ ID NO: 2 or a conservative variant polypeptide thereof, N-terminal amino acids of transmembrane region are partially truncated (eg. 5, 10, 15, 18, 20aa or more truncated) or completely truncated; preferably N-terminal amino acids 2nd to 24th are truncated.

In another preferable example, it further comprises a tag added at the N-terminus; preferably, the tag comprises (but not limited to): 2B1, 17α, MBP; more preferably the tag is 2B1.

In another preferable example, the conservative variant polypeptide of SEQ ID NO: 1 or 2 comprises: (1) polypeptides derived from the sequence shown in SEQ ID NO: 1 or 2, being formed by substitution, deletion or addition of one or more (for example 1-20, preferably 1-10, more preferably 1-5, more preferably 1-3) amino acid residues, and have a catalytic function of C-2 position or C-3' position hydroxylation of the flavanone (class) compounds; (2) polypeptides having at least 80% (preferably at least 85%, more preferably at least 90%, more preferably at least 95%, more preferably at least 99%) sequence identity to the amino acid sequence shown in SEQ ID NO: 1 or 2 and having a catalytic function of C-2 position or C-3' position hydroxylation of the flavanone (class) compounds; or (3) polypeptides derived from the sequence shown in SEQ ID NO: 1 or 2 with a tag added at the N-terminus or C-terminus; or, a signal polypeptide fused at the N-terminus.

Another aspect of the present disclosure provides a method of synthesizing flavone C-glycoside (class) compounds or intermediates thereof, comprising: (1) catalyzing flavanone (class) compounds by a novel flavone hydroxylase to form the hydroxylation at the C-2 position or C-3' position; the novel flavone hydroxylase is a polypeptide shown in SEQ ID NO:1 or a conservative variant polypeptide thereof, which catalyzes the hydroxylation of flavanone (class) compounds at the C-2 position; or, the novel flavone hydroxylase is a polypeptide shown in SEQ ID NO:2 or a conservative variant polypeptide thereof, which catalyzes hydroxylation of flavanone (class) compounds at the C-3'position; (2) the product of C-2 position hydroxylation of (1) is C-glycoside glycosylated to obtain C-glycoside-2-hydroxyflavanone (class) compounds; or, the product of C-3' position hydroxylation of (1) is further hydroxylated at C-2 position, then C-glycoside glycosylated to obtain flavone C-glycoside (class) compounds (C-glycoside-2,3'-dihydroxyflavanone compounds) or intermediates thereof.

In another preferable example, the C-glycoside glycosylation is carried out by C-glycoside glycosyltransferases.

In another preferable example, prior to (1), it further comprises: (b) a malonyl-CoA structural analogue (for example comprising the form of substitution of one or more groups) and a p-coumaroyl-CoA structural analogue (for example comprising the form of substitution of one or more groups) are catalyzed by chalcone synthase (CHS) and chalcone isomerase (CHI) to obtain flavanone (class) compounds.

In another preferable example, prior to (b), it further comprises: (a) aromatic amino acids are catalyzed by tyrosine ammonia lyase (TAL), or phenylalanine ammonia lyase (PAL) and 4-coumaroyl-CoA ligase to obtain p-coumaroyl-CoA structural analogue.

In another preferable example, the flavanone (class) compound comprises (but not limited to): naringenin, eriodictyol.

In another preferable example, the malonyl-CoA structural analogue comprises (but not limited to): malonyl-CoA or methylmalonyl-CoA.

In another preferable example, the p-coumaroyl-CoA structural analogue comprises (but not limited to): p-coumaroyl-CoA or p-cinnamyl-CoA.

In another preferable example, the aromatic amino acids comprise (but not limited to): L-tyrosine or L-phenylalanine.

In another preferable example, the C-glycoside glycosylation is carried out with C-glycosyltransferase; preferably, the C-glycosyltransferase comprises PhCGT1, OsCGT or ZmCGT.

Another aspect of present disclosure provides a method of biosynthesizing flavanone (class) compounds, wherein, it comprises: co-transferring precursor genes for the synthesis of flavanone (class) compounds and genes encoding a novel flavone hydroxylase into host cells; the novel flavone hydroxylase is a polypeptide shown in SEQ ID NO:1 or a conservative variant polypeptide thereof, which catalyzes the hydroxylation of flavanone compounds in the C-2 position to obtain 2-hydroxyflavanone compounds; and/or, the novel flavone hydroxylase is a polypeptide shown in SEQ ID NO:2 or a conservative variant polypeptide thereof, which catalyzes the hydroxylation of flavanone compounds at the C-3' position to obtain a 3'-hydroxyflavanone compound.

Another aspect of present disclosure provides a method for biosynthesizing flavone C-glycoside (class) compounds or intermediates thereof, comprises: (i) co-transferring precursor genes for the synthesis of flavanone (class) compounds, genes encoding novel flavone hydroxylases and genes encoding C-glycosyltransferases into host cells; the novel flavone hydroxylase is a polypeptide shown in SEQ ID NO: 1 or a conservative variant polypeptide thereof, which catalyzes the hydroxylation of flavanone (class) compounds at the C-2 position; and/or, the novel flavone hydroxylase is a polypeptide shown in SEQ ID NO: 2 or a conservative variant polypeptide thereof, which catalyzes hydroxylation of flavanone (class) compounds at the C-3' position; (ii) culturing cells of (i), thereby biosynthesizing flavone C-glycoside (class) compounds or intermediates thereof.

Another aspect of the present disclosure provides a genetically engineered cell, wherein, it comprises: precursor genes for the synthesis of flavanone (class) compounds and genes encoding novel flavone hydroxylases; wherein, the novel flavone hydroxylase is a polypeptide shown in SEQ ID NO: 1 or a conservative variant polypeptide thereof, which catalyzes the hydroxylation of flavanone (class) compounds at the C-2 position; or, the novel flavone hydroxylase is a polypeptide shown in SEQ ID NO: 2 or a conservative variant polypeptide thereof, which catalyzes hydroxylation of flavanone (class) compounds at the C-3' position.

In one preferable example, the genetically engineered cell also comprises: genes encoding C-glycosyltransferases.

Another aspect of the present disclosure provides a method for preparing the cells mentioned above, wherein it comprises: co-transferring precursor genes for the synthesis of flavanone (class) compounds and genes encoding novel flavone hydroxylases into host cells; wherein, the novel flavone hydroxylase is a polypeptide shown in SEQ ID NO:1 or a conservative variant polypeptide thereof, which catalyzes hydroxylation of flavanone (class) compounds at the C-2-position; or, the novel flavone hydroxylase is a polypeptide shown in SEQ ID NO: 2 or a conservative variant polypeptide thereof, which catalyzes hydroxylation of flavanone (class) compounds at the C-3'position; preferably, further co-transferring genes encoding C-glycosyltransferases into host cells.

Another aspect of present disclosure provides a kit for biosynthesizing flavone C-glycoside (class) compounds or intermediates thereof, wherein it comprises: novel flavone hydroxylases; precursor genes for the synthesis of flavanone (class) compounds; wherein the novel flavone hydroxylase is a polypeptide shown in SEQ ID NO: 1 or a conservative variant polypeptide thereof, which catalyzes the hydroxylation of flavanone (class) compounds at the C-2 position; and/or, the novel flavone hydroxylase is a polypeptide shown in SEQ ID NO: 2 or a conservative variant polypeptide thereof, which catalyzes hydroxylation of flavanone (class) compounds at the C-3' position; preferably, it further comprises C-glycosyltransferases; preferably, it further comprises host cells

Another aspect of present disclosure provides a kit for biosynthesizing flavone C-glycoside (class) compounds or intermediates thereof, comprising the genetically engineered cells mentioned above.

In another preferable example, in the polypeptide shown in SEQ ID NO: 1 or the conservative variant polypeptide thereof, N-terminal amino acids of transmembrane region are partially or completely truncated; preferably N-terminal amino acids 2nd to 24th are truncated; or in the polypeptide shown in SEQ ID NO: 2 or the conservative variant polypeptide thereof, N-terminal amino acids of transmembrane region are partially or completely truncated; preferably N-terminal amino acids 2nd to 24th are truncated; preferably it further comprises a tag added at the N-terminus; more preferably, the tag comprises (but not limited to): 2B1, 17α, MBP; more preferably the tag is 2B1.

In another preferable example, the host cells comprise: prokaryotic cells or eukaryotic cells; preferably, the prokaryotic host cells comprise *Escherichia coli* cells or *Streptomyces* cells, the eukaryotic host cells comprise yeast cells.

In another preferable example, the flavone C-glycoside (class) compounds comprise: oriention, isooriention, vitexin, isovitexin.

In another preferable example, the intermediates of the flavone C-glycoside (class) compounds comprise: 2-hydroxynaringenin-C-glucoside, 2-hydroxyeriodictyol-C-glucoside.

In another preferable example, the precursor genes that synthesizes flavanone (class) compounds comprise genes of aromatic amino acids tyrosine ammonia lyase (TAL) or phenylalanine ammonia lyase (PAL), 4-coumaroyl-CoA ligase, chalcone synthase (CHS), chalcone isomerase (CHI).

In another preferable example, the cells also comprise genes that synthesize glycosyl donors.

In another preferable example, the cells further comprise a cytochrome P450 reductase (for example CPR) expression cassette.

Other aspects of the disclosure will be apparent to those skilled in the art based on the disclosure herein.

### Brief description of the drawings

FIG. 1 Schematic of predicted biosynthetic pathway of 2-hydroxynaringenin.
FIG.2 Schematic of each expression plasmid construction in examples.
FIG.3 (Left) HPLC results of the fermentation broth products of engineering strains sSYW80 and sSYW81. (Right) Yield of 2-hydroxynaringenin produced by engineering strains sSYW80 and sSYW81.
FIG 4. Schematic of predicted biosynthetic pathway of glycosylation products of vitexin, isovitexin and 2-hydroxynaringenin.
FIG.5 (Left) HPLC results of the fermentation broth products of engineering strains sCZ4 and sCZ89. (Right) Yield of products before and after acid treatments of reaction solutions.
FIG.6 Schematic of predicted biosynthetic pathway of eriodictyol.
FIG.7 (Left) HPLC results of the fermentation broth products of engineering strains sCZ51 and sCZ97. (Right) Yield of eriodictyol produced by engineering strains sCZ51 and sCZ97.
FIG.8 Schematic of predicted biosynthetic pathway of glycosylation products of orientin, isoorientin and 2-hydroxyeriodictyol.
FIG.9 (Left) HPLC results of the fermentation broth products of engineering strains sSYW82 and sSYW83. (Right) Yield of products before and after acid treatments of reaction solutions.

### Detailed Description

The inventors are committed to the biosynthesis of flavanone compounds or C-glycoside flavone compounds (flavone C-glycoside compounds), by genomics mining of plants, novel flavone hydroxylases PhF2H and PhF3 'H were obtained, which belong to cytochrome P450 hydroxylases and have the function of hydroxylation of specific positions of the compounds. Further, by modifying the enzymes, using a C-glycoside glycosyltransferase and assembling a synthesis pathway of a flavone precursor, the inventors efficiently synthesize flavone C-glycoside compounds such as orientin, isoorientin, vitexin, isovitexin and related intermediates such as 2-hydroxynaringenin and eriodictyol in the synthesis pathway thereof.

### Bioactive polypeptides, encoding genes, vectors and hosts thereof

By genomics and transcriptome mining, combined with a large number of research and experimental works, the inventors revealed novel flavone hydroxylases PhF2H and PhF3'H. The finding of the inventors in heterologous (exogenous) expressions of the enzyme shows that PhF2H can efficiently catalyze the hydroxylation of flavones such as naringenin and its derivatives at the C-2 position; PhF3'H can efficiently catalyze the hydroxylation of flavones such as naringenin and its derivatives at the C-3' position. Preferably, flavone hydroxylases of the present disclosure are derived from *Gramineae (Monocotyledoneae)*; more preferably, they are derived from *phyllostachys edulis* (or *Phyllostachys heterocycla; Ph).*

The PhF2H has an amino acid sequence shown in SEQ ID NO: 1. The PhF3'H has an amino acid sequence shown in SEQ ID NO: 2.

The present disclosure also includes conservative variant polypeptides of the flavone hydroxylases PhF2H (flavanone-2-hydroxylase) and PhF3'H (flavanone-3'-hydroxylase). In the present disclosure, the "conservative variant polypeptide (s)" refers to a polypeptide that basically maintains same biological functions or activities as the polypeptide described. The "conservative variant polypeptide(s)" may be (i) polypeptides with one or more conservative or non-conservative amino acid substitution (preferably conservative), where the substituted amino acid residues may or may not be one encoded by the genetic code, or (ii) polypeptides containing substitutions of one or more amino acid residues having a substituent group, or (iii) polypeptides formed having the mature polypeptide fused with another compound (such as compounds that extend half-life of the polypeptide, for example, polyethylene glycol), or (iv) polypeptides formed by having said polypeptide fused with additional amino acid sequence (such as leader sequence or secretory sequence, or sequence used for purification of the polypeptide or proprotein sequence, or fusion protein formed with fragment of antigen IgG). In accordance with the teachings provided herein, these fragments, derivatives and analogs are well known to a person skilled in the art.

The "conservative variant polypeptide" may include but are not limited to: deletion, insertion and/or substitution of one or more (usually 1-50, preferably 1-30, more preferably 1-20, most preferably 1-10) amino acids, and addition or deletion of one or several (usually within 50, preferably within 20 or 10, more preferably within 5) amino acids at the C-terminal and/or N-terminal. For example, substitution with amino acids of comparable or similar properties usually does not change protein function in the art. As another example, addition of one or more amino acids to the C-terminus and/or N-terminus usually does not change the function of a protein either. The present disclosure also provides analogs of the polypeptides. The differences between analogs and the original polypeptide may be the difference in amino acid sequences, and may also be the difference in the forms of modifications that will not affect the sequence, or both. These polypeptides include natural or induced genetic variants. Induced variants can be obtained by a variety of techniques, such as generating random mutagenesis by irradiation or exposure to mutagens, and can also be obtained by directed mutagenesis or other known molecular biology techniques. Analogs mentioned herein also include analogs with residue(s) different from natural L-amino acid (e.g., D-amino acids), as well as analogs with a non-naturally occurred or synthetic amino acid (e.g., β, γ-amino acids). It should be understood that the polypeptides of the present disclosure are not limited to the representative polypeptides described above.

In a preferable embodiment of the disclosure, polypeptide fragments in N-terminal transmembrane region of the flavanone-2-hydroxylase and flavanone-3'-hydroxylase of the present disclosure are removed. The inventors found that removing the N-terminal transmembrane region can increase the soluble expression of the truncated protein, and when it is applied to the biosynthetic pathway, it can exert better activities.

The N-terminal or C-terminal of the flavone hydroxylase of the present disclosure can also comprise one or more polypeptide fragments as protein tag(s). For example, the tag may be FLAG, HA, HA1, c-Myc, Poly-His, Poly-Arg, Strep-TagII, AU1, EE, T7, 4A6, ε, B, gE, and Ty1. In a preferable embodiment of the disclosure, the tag comprises 2B, 17α, MBP, etc.; more preferably, the tag is 2B1.

When producing the flavone hydroxylase or other enzymes of the present disclosure, in order to make translated proteins be secreted and expressed (such as secreted to the outside of cells), a secretion signal sequence may also be added to the N-terminus of the polypeptide of the present disclosure. The signal peptide can be cut off during the secretion of the polypeptide from cells.

The bioactive polypeptides of the disclosure can be recombinant polypeptides, natural polypeptides, or synthetic polypeptides. The polypeptide of the present disclosure can be a naturally purified product, or a chemically synthesized product, or can be produced by prokaryotic or eukaryotic hosts (for example, bacteria, yeast, higher plants) using recombinant technology. Depending on the host used in the recombinant production, the polypeptide of the present disclosure may be glycosylated or non-glycosylated. The polypeptide of the present disclosure may include or not include the initial methionine residue.

The polynucleotide sequences encoding the C-glycoside flavone hydroxylase and other enzymes of the present disclosure can be in the form of DNA or RNA. Forms of DNA include cDNA, genomic DNA or artificially synthesized DNA. DNA can be single-stranded or double-stranded. The DNA can be a coding strand or a non-coding strand. The term "polynucleotide encoding a/the polypeptide" may include a polynucleotide encoding the polypeptide, or a polynucleotide that further includes additional coding and/or non-coding sequences.

The present disclosure also relates to vectors containing the polynucleotides of the present disclosure, as well as host cells produced by genetic engineering methods using the vectors or coding sequences of the polypeptides of the present disclosure, and methods for producing the polypeptides of the present disclosure through recombinant technology.

Through conventional recombinant DNA technology, recombinant polypeptides can be expressed or produced. Generally speaking, steps are as follows: (1). Transforming or transducing an appropriate host cell with a polynucleotide encoding the polypeptide (including its conservative variant polypeptides), or with a recombinant expression vector containing the polynucleotide; 2). Culturing the host cells in a suitable medium; (3). Isolating and purifying proteins from the culture medium or cells.

In the present disclosure, the polynucleotide sequence encoding the polypeptide can be inserted into a recombinant expression vector. The term "recombinant expression vector" refers to bacterial plasmids, phages, yeast plasmids, plant cell viruses, mammalian cell viruses such as adenovirus, retrovirus or other vectors well known in the art. Any plasmid and vector can be used as long as it can replicate and be stable in the hosts. An important characterastic of an expression vector is that it usually contains an origin of replication, a promoter, a marker gene, and translation control elements. Preferably, the expression vector may be a prokaryotic expression vector.

Suitable methods for constructing expression vector which comprises the polynucleotide encoding the flavone hydroxylase or other enzymes of the present disclosure and appropriate transcriptional/translational control signals are well known to the person skilled in the art. These methods include *in vitro* recombinant DNA technology, DNA synthesis technology, *in vivo* recombinant technology and so on. Said DNA sequence may be effectively linked to a proper promoter in the expression vector to direct mRNA synthesis. In addition, the expression vector preferably contains one or more selective marker genes to provide phenotypic traits for the selection of transformed host cells.

Vectors containing the above appropriate DNA sequences and appropriate promoters or regulatory sequences can be used to transform appropriate host cells so that they can express proteins. The host cell can be a prokaryotic cell, such as a bacterial cell; or a lower eukaryotic cell, such as a yeast cell; or a higher eukaryotic cell, such as a mammalian cell. Representative examples include: bacterial cells of *Escherichia coli, Streptomyces, Bacillus subtilis; Salmonella typhimurium;* fungal cells such as yeast cells, plant cells, Ganoderma lucidum cells; insect cells of Drosophila S2 or Sf9; CHO, COS, 293 cells, or animal cells of Bowes melanoma cells, etc.

The present disclosure also provides host cells for the biosynthesis of flavone C-glycoside compounds or intermediates thereof, wherein it comprises: precursor genes of synthetic flavanone compounds, genes of present disclosure encoding flavanone-2-hydroxylase and/or flavanone-3'-hydroxylase and genes encoding C-glycosyltransferases.

In a preferable embodiment of the disclosure, the host cells is prokaryotic cells; preferably *Escherichia coli* cells, yeast cells or *Streptomyces* cells, more preferably *Escherichia coli* cells. The host cell is a type of production tools. Those skilled in the art can modify other host cells except for *E. coli* by some technical means, so as to also realize the biosynthesis of the present disclosure. The modified host cells and preparation methods should also be included in the present disclosure.

In a preferable embodiment of the disclosure, in the host cells, the precursor genes for the synthesis of flavanone (class) compounds comprise genes of aromatic amino acids tyrosine ammonia lyase (TAL) or phenylalanine ammonia lyase (PAL), 4-coumaroyl-CoA ligase, chalcone synthase (CHS), chalcone isomerase (CHI). The present disclosure also comprises conservative variant polypeptides of the chalcone synthase (CHS), chalcone isomerase (CHI), aromatic amino acids tyrosine ammonia lyase (TAL), phenylalanine ammonia lyase (PAL), 4-coumaroyl-CoA ligase, C-glycosyltransferases, and so on.

In a preferable embodiment of the disclosure, the cells also comprise genes that synthesize glycosyl donors. The glycosyl group comprises glucose; preferably, the glycosyl donor is a compound carrying a glucose group; for example, the glycosyl donor comprises UDP glucose.

In a preferable embodiment of the disclosure, the cells further comprise a cytochrome P450 reductase expression cassette, which is used in combination with the cytochrome P450 hydroxylase of the present disclosure to provide reduction. The present disclosure also includes conservative variant polypeptides of the cytochrome P450 reductase.

### Use and preparation methods

The flavone hydroxylates PhF2H and PhF3'H or their conservative variant polypeptides of the present disclosure can be applied to specifically and efficiently catalyze the hydroxylation of the C-2 or C-3' positions of flavanone compounds to produce a type of products hydroxylating at specific positions, combined with glycosylation of C-glycosides to further obtain C-glycoside-2-hydroxyflavanone compounds or C-glycoside-2,3'-dihydroxyflavanone compounds, and by further dehydration reaction to form flavone C-glycoside compounds.

Therefore, the present disclosure provides a use of the flavone hydroxylases PhF2H and PhF3'H or their conservative variant polypeptides for catalyzing C-2 or C-3' position hydroxylation of flavanone compounds; wherein, the flavone hydroxylase PhF2H is a polypeptide shown in SEQ ID NO: 1 or its conservative variant polypeptides; the flavone hydroxylase PhF3'H is a polypeptide shown in SEQ ID NO: 2 or its conservative variant polypeptides.

As used herein, the 2-hydroxy flavanone compounds comprise 2-hydroxy flavanone or its derivatives, structural analogues and isomers. The C-glycoside-2-hydroxyflavanone compounds comprise C-glycoside-2-hydroxyflavanone or its derivatives, structural analogues and isomers. The flavone C-glycoside compounds comprise flavone C-glycoside or its derivatives, structural analogues and isomers.

In a preferable embodiment of the disclosure, the flavanone compounds comprise: naringenin, eriodictyol, pinocembrin, hesperidin, sakuranetin. In addition, their analogues or variants with substituted groups should also be included.

In a preferable embodiment of the disclosure, the 2-hydroxy flavanone compounds comprise: 2-hydroxynaringenin, 2-hydroxyeriodictyol; and/or the C-glycoside-2-hydroxy flavanone compounds comprise: 2-hydroxynaringenin-6-C(8-C)-glucoside, 2-hydroxyeriodictyol-6-C(8-C)-glucoside. In addition, their analogues or variants with substituted groups should also be included.

During the glycosylation of C-glycosides, UDP glucose can be used as the glycosyl donor. It should be understood that other compounds carrying active glycosyl groups can also be used as donors and should also be included in the present disclosure.

The present disclosure also provides a method for catalyzing C-2 position or C-3' position hydroxylation of flavanone compounds, comprising: catalyzing with a novel flavone hydroxylase; wherein the novel flavone hydroxylase is a polypeptide shown in SEQ ID NO: 1 or a conservative variant polypeptide thereof, which catalyzes the C-2 hydroxylation of flavanone compounds; or wherein the novel flavone hydroxylase is a polypeptide shown in SEQ ID NO:2 or a conservative variant polypeptide thereof, which catalyzes the hydroxylation of flavanone compounds at the C-3'position.

The present disclosure also provides a method of synthesizing flavone C-glycoside compounds, comprising:(1) catalyzing flavanone compounds by a novel flavone hydroxylase to induce hydroxylation at the C-2 position or C-3' position; the novel flavone hydroxylase is a polypeptide shown in SEQ ID NO:1 or a conservative variant polypeptide thereof, which catalyzes the hydroxylation of flavanone compounds at the C-2 position; or, the novel flavone hydroxylase is a polypeptide shown in SEQ ID NO:2 or a conservative variant polypeptide thereof, which catalyzes hydroxylation of flavanone compounds at the C-3'position;(2) the product of C-2 position hydroxylation of (1) is C-glycoside glycosylated to obtain C-glycoside-2-hydroxyflavanone compounds; or, the product of C-3' position hydroxylation of (1) is further hydroxylated at the C-2 position, then C-glycoside glycosylated to obtain C-glycoside-2,3'-dihydroxy flavanone (class) compounds. Preferably, prior to catalyzation with the novel flavone hydroxylase, it further comprises: (b) a malonyl-CoA structural analogue and a p-coumaroyl-CoA structural analogue are catalyzed by chalcone synthase (CHS) and chalcone isomerase (CHI) to obtain flavanone (class) compounds. Preferably, prior to (b), it further comprises: (a) aromatic amino acids are catalyzed by tyrosine ammonia lyase (TAL), or phenylalanine ammonia lyase (PAL) and 4-coumaroyl-CoA ligase to obtain p-coumaroyl-CoA structural analogue.

In a preferable embodiment of the disclosure, the malonyl-CoA structural analogue comprises: malonyl-CoA, methylmalonyl-CoA; the p-coumaroyl-CoA structural analogue comprises: p-coumaroyl-CoA or p-cinnamyl- CoA; the L-tyrosine structural analogue comprises: L-tyrosine, L-phenylalanine. It should be understood that according to the overall description of the present disclosure, their analogues or variants can also be applied to the present disclosure.

In a preferable embodiment of the disclosure, the 2-hydroxy flavanone compound is 2-hydroxynaringenin, which is obtained from naringenin through flavanone-2-hydroxylase (F2H) catalysis; or the 2-hydroxy flavanone compound is 2-hydroxyeriodictyol, which is obtained from eriodictyol by flavanone-2-hydroxylase (F2H) catalysis; preferably, the eriodictyol is obtained from naringenin through flavanone-3'-hydroxylase (F3'H) catalysis. It should be understood that according to the overall description of the present disclosure, their analogues or variants can also be applied to the present disclosure.

The present disclosure also provides a method of biosynthesizing flavanone compounds, comprising: co-transferring precursor genes for the synthesis of flavanone compounds and genes encoding a novel flavone hydroxylase into host cells to obtain 2-hydroxyflavanone compounds and/or 3'-hydroxyflavanone compounds.

The present disclosure also provides a method of biosynthesizing flavone C-glycoside compounds, comprising: (i) co-transferring precursor genes for the synthesis of flavanone compounds, genes encoding novel flavone hydroxylases and genes encoding C-glycosyltransferases into host cells; wherein, the novel flavone hydroxylase is a polypeptide shown in SEQ ID NO: 1 or a conservative variant polypeptide thereof, which catalyzes the hydroxylation of flavanone compounds at the C-2 position; and/or, the novel flavone hydroxylase is a polypeptide shown in SEQ ID NO: 2 or a conservative variant polypeptide thereof, which catalyzes hydroxylation of flavanone compounds at the C-3' position; (ii) culturing cells of (i), thereby biosynthesizing C-glycoside flavone compounds.

In specific embodiments of the present disclosure, the inventors found that the biosynthesis system of flavone C-glycoside compounds constructed by the present disclosure has very high product yield, that is, high-efficient biosynthesis is realized.

Compared with traditional plant extraction methods, microbial fermentation has several advantages, such as more fast extraction, less affection by external factors and so on. Also, the yield of some compounds synthesized by microorganisms is much higher than that of plant extraction. Microbial fermentation has become an important method of obtaining natural products. Due to the low natural abundance of flavone C-glycoside compounds and the coexistence with a large number of similar structural flavone glycosides and phenylpropanoids in plant extracts, isolation and purification of flavone C-glycoside compounds are cumbersome and complicated. In the present disclosure, the method of microbial fermentation is used to efficiently and directionally synthesize flavone C-glycoside compounds, which extremely effectively reduces the cost of isolation and purification of such compounds.

The present disclosure also provides a kit for biosynthesizing flavone C-glycoside compounds or intermediates thereof, wherein, it comprises: novel flavone hydroxylases shown in SEQ ID NO: 1-2 or conservative variant polypeptides thereof; C-glycosyltransferases; precursor genes for the synthesis of flavanone (class) compounds; preferably, it further comprises host cells. More preferably, the kit further comprises instructions of biosynthetic methods.

### The main advantages of the present disclosure are:

After a large number of screenings, novel flavone hydroxylases PhF2H and PhF3'H have been obtained. They have functions of hydroxylating the C-2 or C-3' positions of flavanone compounds, and their effects are better than that of OsF2H, OsF3'H with known functions. The inventor also optimized the two enzymes, which effectively improved their expression efficiency.

Using the novel flavone hydroxylase (especially optimized and modified) and other enzymes, with host cells modified by genetic engineering methods, engineering strains of high-yield flavone C-glycoside compounds such as oriention, isooriention, vitexin and isovitexin are obtained.

The disclosure if further illustrated by the specific examples described below. It should be understood that these examples are merely illustrative, and do not limit the scope of the present disclosure. The experimental methods without specifying the specific conditions in the following examples generally used the conventional conditions, such as those described in J. Sambrook, Molecular Cloning: A Laboratory Manual (3rd ed. Science Press, 2002) or followed the manufacturer's recommendation.

### Experimental Materials

*Phyllostachys edulis* was collected from Shanghai Chenshan Botanical Garden, and rice, sorghum, and corn were all taken from Chinese Academy of Sciences (CAS) center for excellence in molecular plant sciences in Shanghai.

Oligonucleotide primers were purchased from Sangon Biotech and GenScript Biotech Corp.

The synthesized optimized sequence was purchased from GenScript Biotech Corp.

AxyPrep Total RNA Miniprep Kit, PCR Gel Extraction kit, Plasmid Extraction Kit are all from Axygen (American); PrimeScript RT reagent Kit with gDNA Eraser (Perfect Real Time), PrimeSTAR Max DNA Polymerase are from TAKARA. Blunt-end cloning was used by pEASY-Blunt Simple Cloning Kit (Beijing Quanshijin Biotechnology Co., Ltd.).

Plant genomic DNA extraction was used by TIANGEN Plant Genome Extraction Kit.

Restriction endonucleases are all from NEB.

*E. coli* DH10B, BL21(DE3) and pET21a, pET28a vectors were used for gene cloning and protein expression.

Naringenin, eriodictyol, oriention, isooriention, vitexin and isovitexin Standards were purchased from Dalian Meilun Biotechnology Co., Ltd.

Other reagents are analytical grade reagent or chromatographic grade reagent, purchased from Sinopharm Chemical Reagent Co., Ltd.

PCR was conducted on Arktik Thermal Cycler (Thermo Fisher Scientific).

Dionex UltiMate 3000 Liquid Chromatography System (Thermo Fisher Scientific) was used for HPLC.

HRMS was measured by the Thermo Fisher Scientific LTQ-Orbitrap combined with MS Q Exactive.

### Example 1. Novel cytochrome P450 hydroxylase

In present disclosure, by mining the genomic information of *Phyllostachys edulis,* two novel cytochrome P450 hydroxylases were obtained, the amino acid sequences of which are as follows:
>PhF2H (SEQ ID NO: 1; underline is the N-terminal transmembrane region) 
MAMELVALVLAAMLLLTVIMRRYGGSAGSSKHGRLPPSPMALPFIGHLHLIRPPPHRAFDRIIA
RHGPLIYLRLGPSTHCVVVGSADVARDFLRFEASIPERPLTAVTRQLAYGSAGFAFAPYGPHWRFMK
RLCMSELLGPRTVEQLRPVRDAELAGVLRAVRAAAERGESVDMSRELIRMSNNAIMRMVASALPGD
MAEAARDCAKQVAELVGAFNVEDYVAMCRGWDLQGIGRRTREVHARFDALLETMIRAKEEARRDP
LPRDKSRTTKDLLDILMDVAEDEAAEVRLTRENIKAFVLDIFTAGSDTTATSVEWMLAELINHPAYLE
KLRAEVDAVVGGSRLVGEPDVAQMPYLQAVLKETLRLRPPAVFAQRETIEAVHVCGYTIPPKTSVFF
NIFSIGRDPACWEDPLEFRPERFMPGGASAGVDPKGQHQQLMPFGSGRRACPGMGLAMQAVPAFLA
ALVQCFDWAVPLPQGTPLDMEEAEGLVSARKQPLVLVPTQRLRPLPAGAAAALA*
>PhF3'H (SEQ ID NO: 2; underline is the N-terminal transmembrane region)

MGDVPLPLLLGSLAVSALVWYVLFRRGGGEGAKRPPGPRGWPVLGNLPQLGAKPHHTMCAM
AREYGPLFRLRFGSAEVVVAASAGVAAQFLRGLDANFSNRPPNSGAEHVAYNYQDLVFAPYGARW
RALRKLCALHLFSAKALDDLRAVREGEVALLVRELARAAAGGVPAVALGQEANVTATNTLARATV
GRRVFAVDGGEGAREFKEMVVELMQLAGVFNVGDFVPALRWLDPQGVVAKMKRLHRRYDDMLN
GFIKERKAVVKPDGLSGGDQGKDLLSVLLARMREEQPLDGEDGKITETDIKALLLNLFTAGTDTTSST
VEWALAELIRHPDVLKQAQHELDAVAGRDRLVSESDLPRLTFLAAVIKETFRLHPSTPLSLPRVAAEE
CTVDGYRVPKGTTLLVNVWAIARDPASWPNPLDFNPARFLPGGTHESVDLKGSDFELIPFGAGRRVC
AGLSWGLRMVTLMTATLVHAFDWALVDGMTPDKLNMEEAYGLTLQRAVPLMVQPAPRLLPSAYG
M*

The above two novel cytochrome P450 hydroxylases were used to perform *in vivo* functional verification based on a recombinant microbial system in the subsequent Examples 2 and 3.

### Example 2. Production of 2-hydroxynaringenin by using cytochrome P450 hydroxylase PhF2H

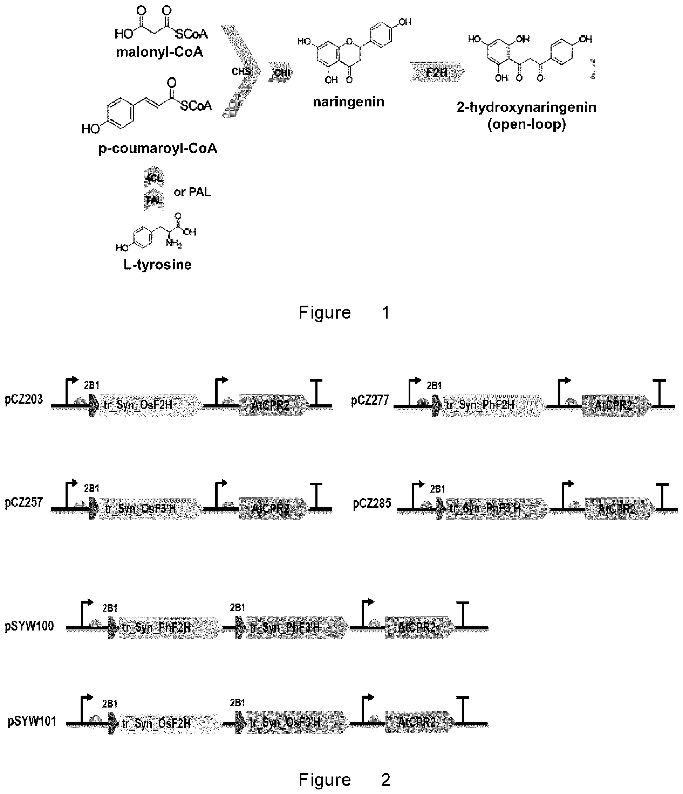

In this example, predicted biosynthetic pathway of 2-hydroxynaringenin is shown in Figure 1. It mainly comprises: after L-tyrosine catalyzed by tyrosine ammonia lyase (TAL) or phenylalanine ammonia lyase (PAL) and 4-coumaroyl-CoA ligase (4CL), the obtained p-coumaroyl-CoA was catalyzed by chalcone synthase (CHS) and chalcone isomerase (CHI) to obtain naringenin, then further catalyzed by flavanone-2-hydroxylase (F2H) to obtain 2-hydroxynaringenin (open-loop).

### 1. Construction of a plasmid for de novo synthesis of naringenin

The artificially synthesized and codon-optimized (*E. coli* preference) precursor gene sequence was constructed into the pET28a vector to obtain pET28-RtPAL, pET28-Pc4CL, pET28-PxhCHS and pET28a-MsCHI, respectively. Among them, PxhCHS: GenBank Accession No. KF765781; MsCHI: GenBank Accession No. KF765782; Pc4CL: GenBank Accession No. KF765780; RtPAL: GenBank Accession No. AAA33883. PCR primers are shown in Table 1.

*Pc4CL* fragment was amplified using pET28-Pc4CL as template and 4CL-F-NcoI/4CL-R-BamHI as primers. pYH40 was obtained by ligation of the amplified products with *Nco*I/*BamH*I digested pCDFDuet-1.

*PxhCHS* fragment was amplified using pET28-PxhCHS as template and CHS-F-NdeI/CHS-R-XhoI as primers. pYH50 was obtained by ligation of the amplified products with *Nde*I/*Xho*I digested pYH40.

*McCHI* fragment was amplified using pET28-MsCHI as template and T7CHI-F-XhoI/CHI-R-AvrII as primers. pYH51 was obtained by ligation of the amplified products with *Xho*I/*Avr*II digested pYH50.

*RtPAL* fragment was amplified using pET28-RtPAL as template and T7PAL-F-BamHI/PAL-R-HindIII as primers. pYH55 obtained by ligation of the amplified products with *BamH*I/*Hind*III digested pYH51 was used to de novo synthesis of naringenin.

**Table 1. Primers used in the construction of expression cassettes with precursor genes for the synthesis of naringenin**

| Primers | Sequences (5'→3') |
|---|---|
| 4CL-F-NcoI | tataccatgggtgactgcgttgccccg(SEQ ID NO: 3) |
| 4CL-R-BamHI | cgggatccttacttcggcaggtcgccgctc(SEQ ID NO: 4) |
| T7PAL-F-BamHI | cgggatcccttatgcgactcctgcattag(SEQ ID NO: 5) |
| PAL-R-HindIII | gcccaagcttttatgccagcatcttc(SEQ ID NO: 6) |
| CHS-F-NdeI | agatatacatatggttacgtggaagaatac(SEQ ID NO: 7) |
| CHS-R-XhoI | ccgctcgagttaggtagccacactatgcag(SEQ ID NO: 8) |
| T7CHI-F-XhoI | ccgctcgagctagaaataattttgtttaac(SEQ ID NO: 9) |
| CHI-R-AvrII | gagcctaggttagttaccgattttaaag(SEQ ID NO: 10) |

### 2. Optimization of PhF2H polypeptide sequence and construction of PhF2H/CPR expression cassette

Codon optimized coding gene (*E. coli* preference) of flavanone-2-hydroxylase *PhF2H* is suitable for the expression in *E. coli.* The codon-optimized sequence of *PhF2H* was modified at the N-terminal of the protein by truncating the N-terminal transmembrane region (positions 2-24), and adding a 2B1 tag (MAKKTSSKGKLPPGPS) before the first amino acid M. This fragment was cloned into the blunt-ended pEASY-Blunt Simple Cloning Vector.A primer pair CZ277-F/CZ277-R (see Table 2) was used to amplify the optimized PhF2H fragment. The fragment was inserted into the site between *Nco*I and *BamH*I in the multiple cloning site 1 of the pDuet-1 vector by seamless cloning. *AtCPR2* (GenBank Accession No. NM_179141.2) was amplified using primer pair InfuNde-AtCPR2-F/InfuXho-AtCPR2-R (see Table 2), then was inserted into the site between *Nde*I and *Xho*I in the multiple cloning site 2 of the pDuet-1 vector. Therefore, pCZ277 was constructed (Figure 2).

In addition, as a positive control, rice OsF2H (GenBank Accession No. XP_015642954.1, optimized for *E*. *coli* preferred codons) was truncated at the N-terminal transmembrane region (positions 2-26), with a 2B1 tag added before the first amino acid M. pCZ203 was constituted by linking the optimized OsF2H and AtCPR2 into the pDuet-1 vector in the same way (Figure 2). The primer pair was CZ203-F/CZ203-R (see Table 2).

**Table 2. Primers used in the construction of pCZ203 and pCZ277**

| Gene | Primers | Sequences (5'→3') |
|---|---|---|
| *OsF2H* | CZ203-F | ctcccaccaggacctagcatgcgtagcgcgggtagccg(SEQ ID NO: 11) |
| | CZ203-R | tcgaattcggatccactagtttagctataaaagctcggcagcg(SEQ ID NO: 12) |
| *PhF2H* | CZ277-F | agctcccaccaggacctagcatgggcagcgcggg(SEQ ID NO: 13) |
| | CZ277-R | tcgaattcggatccactagtttacgccagcgccg (SEQ ID NO: 14) |
| *AtCPR2* | InfuNde-AtCP R2-F | ataagaaggagatatacatatgtcctcttcttcttcttcgtcaacc(SEQ ID NO: 15) |
| | InfuXho-AtC PR2-R | ttctttaccagactcgagttaccatacatctctaagatatcttccactcgtttgc(SEQ ID NO: 16) |

### 3. Construction of 2-hydroxynaringenin producing strain

Successfully constructed pCZ203 and pCZ277 were co-transformed with plasmid pYH55 into competent cells *of E. coli* BL21 (DE3) to obtain engineered strains, named sSYW80 and sSYW81, respectively.

The cells were cultured in LB solid medium (containing 100 µg/mL ampicillin, 80 µg/mL spectinomycin) overnight at 37°C. Single colony of sSYW80 and sSYW81 was transferred to a 2mL LB liquid medium (containing 100 µg/mL ampicillin, 80 µg/mL spectinomycin) and incubated overnight. The bacterial fluid was transferred to a new 20mL MOPS liquid medium with antibiotics and incubated at 37°C and 250 r/min until OD₆₀₀ reached 0.5-0.6. The culture was cooled down to 16°C, in a water bath, then IPTG inducer was added at a final concentration of 0.1mM, and 2g/L sterilized tyrosine was added. The mixture was placed at 22°C and cultured for 120h at 220 r/min on the shaker. After completion of the fermentation reaction, 0.5 mL of the fermentation broth was taken out and was mixed with 0.5 mL ethyl acetate, the solution was extracted for 3 times. The organic phase was concentrated, and the resulting residue was dissolved with 100 µL of methanol, wherein 20 µL was used for HPLC analysis.

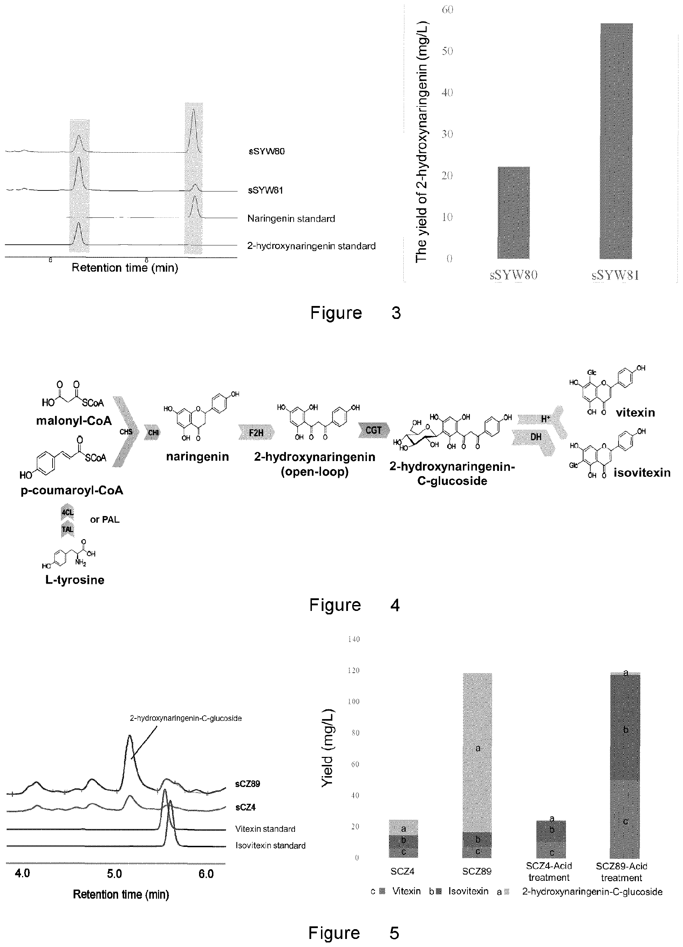

It was detected that the reaction solution contains 2-hydroxynaringenin and unconverted naringenin, as shown in Figure 3 (Left). The retention time of the product is consistent with the product of 2-hydroxynaringenin standard, and the same as the OsF2H product with known functions. This results prove that PhF2H can hydroxylate naringenin at the 2-position. Compared with OsF2H with known functions (corresponding to strain sSYW80, the conversion rate of naringenin is about 35% and the yield of 2-hydroxynaringenin is 22mg/L), using PhF2H strain sSYW81 as the producing strain, the conversion rate of 2-hydroxy naringenin from naringenin was significantly improved, as shown in Figure 3 (Right), which can reach 85%. The production of 2-hydroxynaringenin can reach 57mg/L.

### Example 3. Cytochrome P450 hydroxylase (F2H) combined with C-glycosyltransferase to produce vitexin, isovitexin and their intermediates.

In this example, biosynthetic pathway of glycosylated vitexin, isovitexin, and 2-hydroxynaringenin is shown in FIG. 4. It mainly comprises: after L-tyrosine catalyzed by tyrosine ammonia lyase (TAL) or phenylalanine ammonia lyase (PAL) and 4-coumaroyl-CoA ligase (4CL), the obtained p-coumaroyl-CoA was mixed with malonyl-CoA and catalyzed by chalcone synthase (CHS) and chalcone isomerase (CHI) to obtain naringenin, then further catalyzed by flavanone-2-hydroxylase (F2H) to obtain 2-hydroxynaringenin (open-loop); 2-hydroxynaringenin was converted to 2-hydroxynaringenin-C-glucoside via glycosyltransferase (CGT), then dehydrated into vitexin or isovitexin.

### 1. Construction of a plasmid for de novo synthetic naringenin

The same as in Example 2.

### 2. Construction of optimized F2H/CPR expression cassette

The same as in Example 2.

### 3. Construction of engineered strains and fermentation to produce vitexin, isovitexin and their intermediates.

Construction of plasmid pCZ86 for the glycosylation: target gene PhCGT1 was amplified using *Phyllostachys edulis* genomic DNA as template and Primestar max DNA polymerase as PCR enzyme(primer F: tgccgcgcggcagccatatgatgggccacctggtgc (SEQ ID NO: 17); primer R: tggtgctcgagtgcggccgcctagtccaacactgcaagatccc (SEQ ID NO: 18)). The amplified target band was purified and recovered by agarose gel electrophoresis. pET28a was digested with two restriction endonucleases, *Nde*I/*Not*I, and the PCR target gene PhCGT1 was cloned into the *Nde*I/*Not*I site of pET28a by seamless cloning to obtain pCZ86.

Successfully constructed pCZ203 and pCZ277 were co-transformed with plasmid pYH86 into competent cells *of E. coli* BL21 (DE3) to obtain engineered strains, named sCZ4 and sCZ89, respectively.

The cells were cultured in LB solid medium (containing 100 µg/mL ampicillin, 50µg/mL Kanamycin and 80 µg/mL spectinomycin) overnight at 37°C. Single colony was transferred to a 2mL LB liquid medium (containing 100 µg/mL ampicillin, 50 µg/mL kanamycin and 80 µg/mL spectinomycin) and incubated overnight. The bacterial fluid was transferred to a new 20mL MOPS liquid medium with antibiotics and incubated at 37 °C and 250 r/min until OD₆₀₀ reached 0.5-0.6. The culture was cooled down to 16°C, in a water bath, then IPTG inducer was added at a final concentration of 0.1mM, and 2g/L sterilized tyrosine was added. The mixture was placed at 22°C and cultured for 120h at 220 r/min on the shaker. After completion of the fermentation reaction, 0.5 mL of the fermentation broth was taken out and was mixed with 0.5 mL n-butanol, the solution was extracted for 3 times. The organic phase was concentrated, and the resulting residue was dissolved with 100 µL of methanol, wherein 20 µL was used for HPLC analysis. It was detected that the reaction solution contains vitexin, isovitexin and 2-hydroxynaringenin-C-glucoside, as shown in Figure 5 (Left). As an intermediate, 2-hydroxynaringenin-C-glucoside is unstable to acid. After acid treatments of fermentation products (HCl 1M, 2h), 2-hydroxynaringenin-C-glucoside can be dehydrated into a mixture of vitexin and isovitexin. Yield of products after acid treatments is shown in Figure 5 (Right). Inventors found that PhF2H has excellent activities, and the yield of vitexin and isovitexin corresponding to sCZ89 in total is about 120 mg/L of fermentation broth, which is significantly higher than rice OsF2H (the yield corresponding to sCZ4 is about 25 mg/L).

### Example 4. Using cytochrome P450 hydroxylase F3'H to produce eriodictyol.

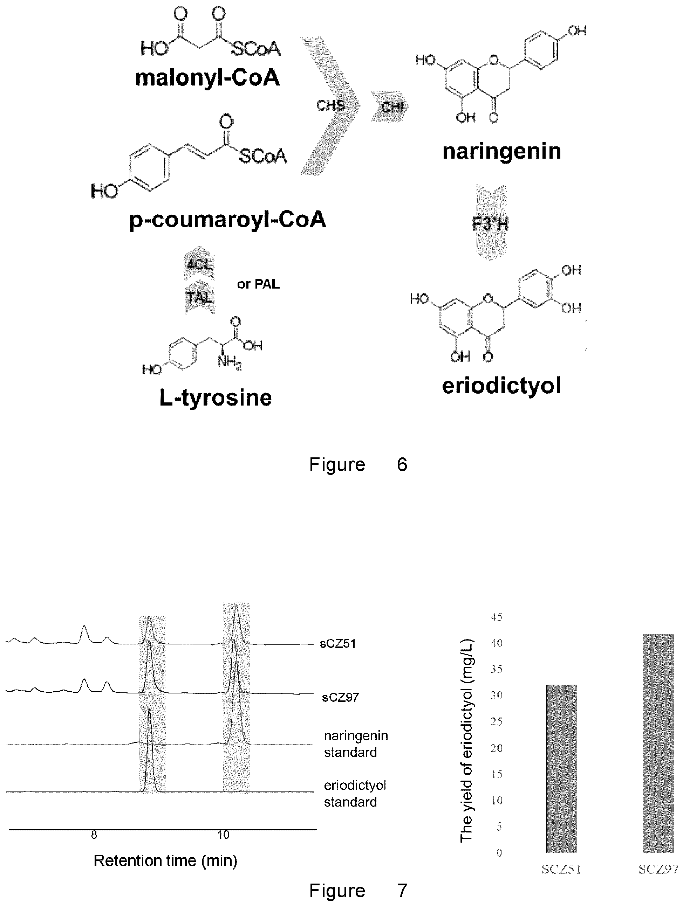

In this example, predicted biosynthetic pathway of eriodictyol is shown in Figure 6. It mainly comprises: after L-tyrosine catalyzed by tyrosine ammonia lyase (TAL) or phenylalanine ammonia lyase (PAL) and 4-coumaroyl-CoA ligase (4CL), the obtained p-coumaroyl-CoA was mixed with malonyl-CoA and catalyzed by chalcone synthase (CHS) and chalcone isomerase (CHI) to obtain naringenin, then further catalyzed by flavanone-3'-hydroxylase (F3'H) to obtain eriodictyol.

### 1. Construction of a plasmid for de novo synthetic naringenin

The same as in Example 2.

### 2. Optimization of PhF3'H and construction of PhF3'H/CPR expression cassette

Codon optimized coding gene (*E. coli* preference) of flavanone-3'-hydroxylase *PhF3'H* is suitable for use in *E. coli.* The codon-optimized sequence of *PhF3'H* was modified at the N-terminal of the protein by truncating the N-terminal transmembrane region (positions 2-24), and adding a 2B1 tag (MAKKTSSKGKLPPGPS(SEQ ID NO: 19)) before the first amino acid M. This fragment was cloned into the blunt-ended pEASY-Blunt Simple Cloning Vector.

Using CZ285-F/CZ285-R as a primer pair (see Table 3), the optimized PhF3'H fragment was amplified by PCR. The amplified fragment was inserted into the site between *Nco*I and *BamH*I in the multiple cloning site 1 of the pDuet-1 vector by seamless cloning. *AtCPR2* was amplified using primer pair InfuNde-AtCPR2-F/InfuXho-AtCPR2-R (see Table 2), then inserted into the site between *Nde*I and *Xho*I in the multiple cloning site 2 of pDuet-1 vector. Therefore, pCZ285 was constructed (Figure 2). As a positive control, rice OsF3'H (GenBank Accession No. XP_015613041.1, optimized for *E. coli* preferred codons for *E. coli*) was truncated at the N-terminal transmembrane region (positions 2-26), with a 2B1 tag added before the first amino acid M. pCZ257 was constituted by linking the optimized OsF3'H and *AtCPR2* into the pDuet-1 vector in the same way (Figure 2). The primer pair was CZ257-F/CZ257-R (see Table 3).

**Table 3. Primers used in the construction of pCZ285 and pCZ257**

| Gene | Primers | Sequences (5'→3') |
|---|---|---|
| *PhF3'H* | CZ285-F | agctcccaccaggacctagcatgcgtcgtggtggcggtga(SEQ ID NO: 20) |
| | CZ285-R | gagctcgaattcggatccactagtttacataccatacgcgctcggc(SEQ ID NO: 21) |
| *OsF3'H* | CZ257-F | agctcccaccaggacctagcatgctgcgtggtggcag(SEQ ID NO: 22) |
| | CZ257-R | gagctcgaattcggatccactagtttaaacgccatacgcgctcg(SEQ ID NO: 23) |

### 3. Construction of eriodictyol producing strain

Successfully constructed pCZ257 and pCZ285 were co-transformed with plasmid pYH55 into competent cells *of E. coli* BL21 (DE3) to obtain engineered strains, named sCZ51 and sCZ97, respectively.

The cells were cultured in LB solid medium (containing 100 µg/mL ampicillin and 80 µg/mL spectinomycin) overnight at 37°C. Single colony of sCZ51 and sCZ97 was transferred to a 2mL LB liquid medium (containing 100 µg/mL ampicillin and 80 µg/mL spectinomycin) and incubated overnight. The bacterial fluid was transferred to a new 20mL MOPS liquid medium with antibiotics and incubated at 37°C and 250 r/min until OD₆₀₀ reached 0.5-0.6. The culture was cooled down to 16°C, in a water bath, then IPTG inducer was added at a final concentration of 0.1mM, and 2g/L sterilized tyrosine was added. The mixture was placed at 22°C and cultured for 120h at 220 r/min on the shaker. After completion of the fermentation reaction, 0.5 mL of the fermentation broth was taken out and was mixed with 0.5 mL ethyl acetate, the solution was extracted for 3 times. The organic phase was concentrated, and the resulting residue was dissolved with 100 µL of methanol, wherein 20 µL was used for HPLC analysis.As shown in Figure 7 (Left), it was detected that the reaction solution contains eriodictyol and unconverted naringenin. The retention time of the product is consistent with the product of eriodictyol standard, and the same as the OsF3'H product. This results prove that OsF3'H can hydroxylate naringenin at the 3'-position. F3'H modified by inventors has excellent activities, as shown in Figure 7 (Right). Its yield of the catalyzation from naringenin to eriodictyol (the conversion rate is 49% and the yield is 41mg/L fermentation broth) is higher than that of rice OsF3 'H (corresponding to sCZ51, the conversion rate is 41% and the yield is 32 mg/L fermentation broth).

### Example 5. Cytochrome P450 hydroxylase (F3'H) combined with C-glycosyltransferase to produce oriention, isooriention and their intermediates

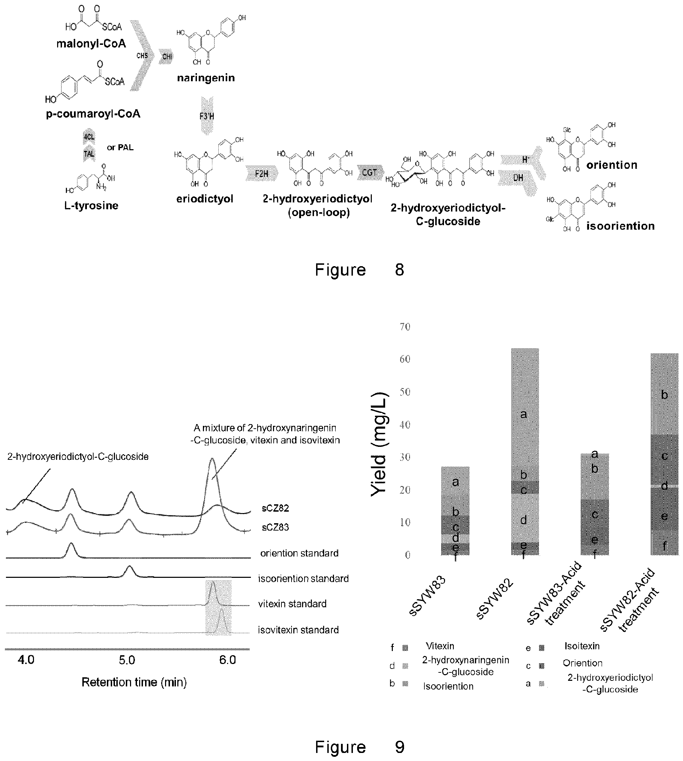

In this example, predicted biosynthetic pathway of glycosylated oriention, isooriention and 2-hydroxyeriodictyol is shown in Figure 8. It mainly comprises: after L-tyrosine catalyzed by tyrosine ammonia lyase (TAL) or phenylalanine ammonia lyase (PAL) and 4-coumaroyl-CoA ligase (4CL), the obtained p-coumaroyl-CoA was mixed with malonyl-CoA and catalyzed by chalcone synthase (CHS) and chalcone isomerase (CHI) to obtain naringenin, further catalyzed by flavanone-3'-hydroxylase (F3'H) to obtain eriodictyol; F2H was used to obtain 2-hydroxyeriodictyol (in an open-loop); 2-hydroxyeriodictyol was converted to 2-hydroxyeriodictyol-C-glucoside via glycosyltransferase (CGT), then dehydrated into oriention or isooriention.

### 1. Construction of a plasmid for de novo synthetic naringenin

The same as in Example 2.

### 2. Construction of optimized F2H/F3'H/CPR binary P450 expression cassette

After pCZ277 digested by *BamH*I/*Not*I, PhF3'H-CPR fragment was amplified by PCR using pCZ285 as templates and SYW100-F/SYW100-R as primers. pSYW100 (containing PhF2H/PhF3'H binary P450 expression cassette) was constructed into the BamHI and NotI sites of pCZ277 by one-step cloning method.

As a positive control, after pCZ203 digested by *BamH*I/*Not*I in the same way, OsF3'H-CPR fragment was amplified by PCR using pCZ257 as templates and SYW101-F/SYW101-R as primers. pSYW101 (containing OsF2H/OsF3'H binary P450 expression cassette) was constructed into the BamHI and NotI sites of pCZ203 by one-step cloning method.

**Table 4. Primers used in the construction of pSYW100 and pSYW101.**

| Gene | Primers | Sequence (5'→3') (SEQ ID NO:) |
|---|---|---|
| *PhF3'H* | SYW100-F | ggcgtaaactagtggatccctctagaaataattttgtttaactttaagaaggagatatac catg(SEQ ID NO: 24) |
| | SYW100-R | cttaagcattatgcggccttacataccatacgcgctcggc(SEQ ID NO: 25) |
| *OsF3'H* | SYW101-F | tagctaaactagtggatccctctagaaataattttgtttaactttaagaaggagatataccatg(SEQ ID NO: 26) |
| | SYW101-R | cttaagcattatgcggccttaaacgccatacgcgctcg(SEQ ID NO: 27) |

### 3. Construction of engineered strains and fermentation to produce oriention, isooriention and their intermediates

The construction plasmid pCZ86 for the glycosylation was the same as in Example 3.

Successfully constructed pSYW100 and pSYW101 were co-transformed with plasmid pYH55 and plasmid pCZ86 into competent cells *of E. coli* BL21 (DE3) to obtain engineered strains, named sSYW82 and sSYW83, respectively.

The cells were cultured in LB solid medium (containing 80 µg/mL spectinomycin, 100 µg/mL ampicillin and 50 µg/mL kanamycin) overnight at 37°C. Single colony was transferred to a 2mL LB liquid medium (containing 80 µg/mL spectinomycin, 100 µg/mL ampicillin and 50 µg/mL kanamycin) and incubated overnight. The bacterial fluid was transferred to a new 20mL MOPS liquid medium with antibiotics and incubated at 37°C and 250 r/min until OD₆₀₀ reached 0.5-0.6. The culture was cooled down to 16°C, in a water bath, then IPTG inducer was added at a final concentration of 0.1mM, and 2g/L sterilized tyrosine was added. The mixture was placed at 22°C and cultured for 120h at 220 r/min on the shaker. After completion of the fermentation reaction, 0.5 mL of the fermentation broth was taken out and was mixed with 0.5 mL n-butanol, the solution was extracted for 3 times. The organic phase was concentrated, and the resulting residue was dissolved with 100 µL of methanol, wherein 20 µL was used for HPLC analysis. The amounts of various products detected in the fermentation broth are shown in the Right panel of Figure 9.

As shown in Figure 9 (Left), a variety of products were detected in the reaction solution, including orientin, isoorientin, vitexin, isovitexin, 2-hydroxynaringenin-C-glucoside and 2-hydroxyeriodictyol-C-glucoside. The yield of products is shown in Figure 9 (Right). As intermediates, 2-hydroxynaringenin-C-glucoside and 2-hydroxyeriodictyol-C-glucoside are unstable to acid. After acid treatments of fermentation products (HCl 1M, 2h), 2-hydroxynaringenin-C-glucoside can be dehydrated into a mixture of vitexin and isovitexin, and 2-hydroxyeriodictyol-C-glucoside can be dehydrated into a mixture of orientin and isoorientin. The yield after acidification is shown in Figure 9 (Right). The combination of PhF2H and PhF3'H modified by inventors has excellent activities. The yield of orientin, isoorientin, vitexin, isovitexin in total from sSYW82 is over 60 mg/L fermentation broth, which is extremely higher than that of rice OsF2H (corresponding to sSYW83, total yield is 30 mg/L).

Each reference provided herein is incorporated by reference to the same extent as if each reference was individually incorporated by reference. In addition, it should be understood that based on the above teaching content of the disclosure, those skilled in the art can practice various changes or modifications to the disclosure, and these equivalent forms also fall within the scope of the appended claims.

## Claims

1. A method of catalyzing C-2 position or C-3' position hydroxylation of flavanone compounds, wherein it comprises: catalyzing with a novel flavone hydroxylase; wherein,
the novel flavone hydroxylase is a polypeptide shown in SEQ ID NO:1 or a conservative variant polypeptide thereof, which catalyzes the hydroxylation of flavanone compounds at the C-2 position; or
the novel flavone hydroxylase is a polypeptide shown in SEQ ID NO:2 or a conservative variant polypeptide thereof, which catalyzes the hydroxylation of flavanone compounds at the C-3'position.

2. The method according to claim 1, wherein the flavanone compound has a parent nucleus structure of formula (I), forms a parent nucleus structure of formula (II) after hydroxylation at the C-2 position, or a parent nucleus structure of formula (III) after hydroxylation at the C-3'position;

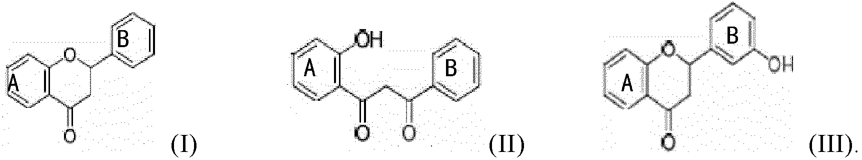

3. The method according to claim 2, wherein, in A ring or B ring, there are 1, 2 or 3 hydroxyl groups.

4. The method according to claim 1, wherein the flavanone compound comprises: naringenin, eriodictyol, pinocembrin, hesperidin, sakuranetin;
the product of hydroxylation at the C-2 position is a 2-hydroxy flavanone compound, comprising: 2-hydroxynaringenin, 2-hydroxyeriodictyol, 2-hydroxypinocembrin, 2-hydroxyhesperidin, 2-hydroxysakuranetin; after hydroxylation at the C-2 position of the flavanone compound, the 2-hydroxy flavanone compound is formed preferably in an open-loop; or
the product of hydroxylation at the C-3' position is a 3'-hydroxy flavanone compound, comprising: eriodictyol, 3'-hydroxyeriodictyol, 3'-hydroxysakuranetin.

5. Use of a novel flavone hydroxylase for catalyzing C-2 or C-3' position hydroxylation of flavanone compounds, the novel flavone hydroxylase is a polypeptide shown in SEQ ID NO:1 or a conservative variant polypeptide thereof, which catalyzes the hydroxylation of flavanone compounds at the C-2 position; or
the novel flavone hydroxylase is a polypeptide shown in SEQ ID NO:2 or a conservative variant polypeptide thereof, which catalyzes the hydroxylation of flavanone compounds at the C-3' position.

6. The use according to claim 5, wherein the flavanone compound has a parent nucleus structure of formula (I), a parent nucleus structure of formula (II) after hydroxylation at the C-2 position, or a parent nucleus structure of formula (III) after hydroxylation at the C-3' position;

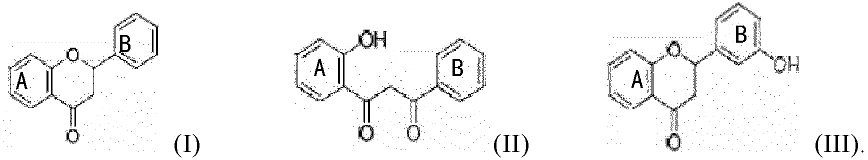

7. The use according to claim 6, wherein the flavanone compound comprises: naringenin, eriodictyol, pinocembrin, hesperidin, sakuranetin;
the product of hydroxylation at the C-2 position comprising: 2-hydroxynaringenin, 2-hydroxyeriodictyol, 2-hydroxypinocembrin, 2-hydroxyhesperidin, 2-hydroxysakuranetin; after hydroxylation at the C-2 position of the flavanone compound, the 2-hydroxy flavanone compound is formed preferably in an open-loop; or
the product of hydroxylation at the C-3' position comprising: eriodictyol, 3'-hydroxyeriodictyol, 3'-hydroxysakuranetin.

8. According to claim 1 or claim 5, wherein, in the polypeptide shown in SEQ ID NO: 1 or a conservative variant polypeptide thereof, N-terminal amino acids of transmembrane region are partially or completely truncated; preferably N-terminal amino acids 2nd to 24th are truncated; or
in the polypeptide shown in SEQ ID NO: 1 or a conservative variant polypeptide thereof, N-terminal amino acids of transmembrane region are partially or completely truncated; preferably N-terminal amino acids 2nd to 24th are truncated.

9. According to claim 8, wherein it further comprises a tag added at the N-terminus; preferably, the tag comprises: 2B1, 17α, MBP; more preferably the tag is 2B1.

10. According to any one of claims 1 to 9, wherein, conservative variant polypeptide of SEQ ID NO: 1 or 2 comprises:
(1) polypeptides derived from the sequence shown in SEQ ID NO: 1 or 2, being formed by substitution, deletion or addition of one or more amino acid residues, and have a catalytic function of C-2 position or C-3' position hydroxylation of the flavanone compounds;
(2) polypeptides having at least 80% sequence identity to the amino acid sequence shown in SEQ ID NO: 1 or 2 and having a catalytic function of C-2 position or C-3' position hydroxylation of the flavanone compounds;
(3) polypeptides derived from the sequence shown in SEQ ID NO: 1 or 2 have a tag added at N-terminus or C-terminus; or, a signal polypeptide fused at N-terminus.

11. A method of synthesizing flavone C-glycoside compounds or intermediates thereof, wherein, it comprises:
(1) catalyzing flavanone compounds by a novel flavone hydroxylase to form the hydroxylation at the C-2 position or C-3' position; the novel flavone hydroxylase is a polypeptide shown in SEQ ID NO:1 or a conservative variant polypeptide thereof, which catalyzes the hydroxylation of flavanone compounds at the C-2 position; or, the novel flavone hydroxylase is a polypeptide shown in SEQ ID NO:2 or a conservative variant polypeptide thereof, which catalyzes hydroxylation of flavanone compounds at the C-3'position;
(2) the product of C-2 position hydroxylation of (1) is C-glycoside glycosylated to obtain C-glycoside-2-hydroxyflavanone compounds; or, the product of C-3' position hydroxylation of (1) is further hydroxylated at the C-2 position, then C-glycoside glycosylated to obtain flavone C-glycoside compounds or intermediates thereof.

12. The method according to claim 11, wherein, prior to (1), it further comprises: (b) a malonyl-CoA structural analogue and a p-coumaroyl-CoA structural analogue are catalyzed by chalcone synthase and chalcone isomerase to obtain flavanone compounds.

13. The method according to claim 12, wherein, prior to (b), it further comprises: (a) aromatic amino acids are catalyzed by tyrosine ammonia lyase or phenylalanine ammonia lyase and 4-coumaroyl-CoA ligase to obtain p-coumaroyl-CoA structural analogue.

14. The method according to claim 11, wherein the flavanone compound comprises: naringenin, eriodictyol;
the malonyl-CoA structural analogue comprising: malonyl-CoA or methylmalonyl-CoA;
the p-coumaroyl-CoA structural analogue comprising: p-coumaroyl-CoA or p-cinnamyl- CoA;
the aromatic amino acids comprise: L-tyrosine or L-phenylalanine; or
the C-glycoside glycosylation is carried out with C-glycosyltransferase; preferably, the C-glycosyltransferase comprises PhCGT1, OsCGT or ZmCGT.

15. A method of biosynthesizing flavanone compounds, wherein, it comprises: co-transferring precursor genes for the synthesis of flavanone compounds and genes encoding a novel flavone hydroxylase into host cells; the novel flavone hydroxylase is a polypeptide shown in SEQ ID NO:1 or a conservative variant polypeptide thereof, which catalyzes the hydroxylation of flavanone compounds at the C-2 position to obtain 2-hydroxyflavanone compounds; and/or, the novel flavone hydroxylase is a polypeptide shown in SEQ ID NO:2 or a conservative variant polypeptide thereof, which catalyzes the hydroxylation of flavanone compounds at the C-3' position to obtain a 3'-hydroxyflavanone compound.

16. A method of biosynthesizing flavone C-glycoside compounds or intermediates thereof, wherein, comprises:
(i) Co-transferring precursor genes for the synthesis of flavanone compounds, genes encoding novel flavone hydroxylases and genes encoding C-glycosyltransferases into host cells; the novel flavone hydroxylase is a polypeptide shown in SEQ ID NO: 1 or a conservative variant polypeptide thereof, which catalyzes the hydroxylation of flavanone compounds at the C-2 position; and/or, the novel flavone hydroxylase is a polypeptide shown in SEQ ID NO: 2 or a conservative variant polypeptide thereof, which catalyzes hydroxylation of flavanone compounds at the C-3' position;
(ii) Culturing cells of (i), thereby biosynthesizing flavones C-glycoside compounds or intermediates thereof.

17. A genetically engineered cell, wherein, it comprises: precursor genes for the synthesis of flavanone compounds and genes encoding novel flavone hydroxylases; wherein, the novel flavone hydroxylase is a polypeptide shown in SEQ ID NO: 1 or a conservative variant polypeptide thereof, which catalyzes the hydroxylation of flavanone compounds at the C-2 position; and/or, the novel flavone hydroxylase is a polypeptide shown in SEQ ID NO: 2 or a conservative variant polypeptide thereof, which catalyzes hydroxylation of flavanone compounds at the C-3' position.

18. The genetically engineered cell according to claim 17, wherein it further comprises: genes encoding C-glycosyltransferases.

19. A method of preparing the cells according to claim 17 or 18, wherein it comprises: co-transferring precursor genes for the synthesis of flavanone compounds, genes encoding novel flavone hydroxylases into host cells; wherein, the novel flavone hydroxylase is a polypeptide shown in SEQ ID NO:1 or a conservative variant polypeptide thereof, which catalyzes hydroxylation of flavanone compounds at the C-2-position; or, the novel flavone hydroxylase is a polypeptide shown in SEQ ID NO: 2 or a conservative variant polypeptide thereof, which catalyzes hydroxylation of flavanone compounds at the C-3'position; preferably, further co-transferring genes encoding C-glycosyltransferases into host cells.

20. A kit for biosynthesizing flavone C-glycoside compounds or intermediates thereof, wherein it comprises: novel flavone hydroxylases; precursor genes for the synthesis offlavanone compounds; wherein the novel flavone hydroxylase is a polypeptide shown in SEQ ID NO: 1 or a conservative variant polypeptide thereof, which catalyzes the hydroxylation of flavanone compounds at the C-2 position; and/or, the novel flavone hydroxylase is a polypeptide shown in SEQ ID NO: 2 or a conservative variant polypeptide thereof, which catalyzes hydroxylation of flavanone compounds at the C-3' position; preferably, it further comprises C-glycosyltransferases; preferably, it further comprises host cells; or
wherein it comprises the genetically engineered cells according to claim 17 or 18.

21. According to any one of the claims 11 to 20, wherein, in the polypeptide shown in SEQ ID NO: 1 or the conservative variant polypeptide thereof, N-terminal amino acids of transmembrane region are partially or completely truncated; preferably N-terminal amino acids 2nd to 24th are truncated; or in the polypeptide shown in SEQ ID NO: 2 or the conservative variant polypeptide thereof, N-terminal amino acids of transmembrane region are partially or completely truncated; preferably N-terminal amino acids 2nd to 24th are truncated; preferably it further comprises a tag added at the N-terminus; more preferably, the tag comprises: 2B1, 17α, MBP; more preferably the tag is 2B1.

22. According to any one of the claims 11 to 20, wherein, the host cells comprise: prokaryotic cells or eukaryotic cells; preferably, the prokaryotic host cells comprise *Escherichia coli* cells or *Streptomyces* cells, the eukaryotic host cells comprise yeast cells.

23. According to any one of the claims 11 to 20, wherein, the flavone C-glycoside compounds comprise: oriention, isooriention, vitexin, isovitexin; or
the intermediates of the flavone C-glycoside compounds comprise: 2-hydroxynaringenin-C-glucoside, 2-hydroxyeriodictyol-C-glucoside.

24. According to any one of the claims 11 to 20, wherein, the precursor genes for the synthesis of flavanone compounds comprise genes of aromatic amino acids tyrosine ammonia lyase or phenylalanine ammonia lyase, 4-coumaroyl-CoA ligase, chalcone synthase, chalcone isomerase; or
the cells also comprise genes that synthesize glycosyl donors; or
the cells further comprise a cytochrome P450 reductase expression cassette.
